# EUROPEAN PATENT APPLICATION

(11) **EP 3 069 800 A1**
(43) Date of publication of application: **21.09.2016**
(21) Application number: 15397512.3
(22) Date of filing: 17.03.2015
(51) Int. Cl.: B08B 15/02, A61L 2/26, A61L 2/08

(54) **BIOSAFETY CABINET AND METHOD OF STERILISING AN OBJECT**

(71) Applicant: Kojair Tech Oy, 35700 Vilppula (FI)
(72) Inventor: Wouters, Arno, 5261 AD Vught (NL)
(74) Representative: Tampereen Patenttitoimisto Oy

(57) **Abstract**

A biosafety cabinet (500) comprises:
- a cavity (110), which has an aperture (120), and
- one or more heating elements (HE1) arranged to heat an object (OBJ1) inserted into the cavity (110) in order to sterilize said object (OBJ1),
wherein the aperture (120) of the cavity (110) is located in the safe operating zone (ZONE1) of the cabinet (500), and the cavity (110) is attached to a wall (210) of the cabinet (500).

## Description

### FIELD

The present invention relates to biosafety cabinets.

### BACKGROUND

A biosafety cabinet provides an enclosed ventilated workspace for safe working with a material, which is contaminated or potentially contaminated with a pathogen. The user of the biosafety cabinet may need to sterilize instruments during the work.

It is known that instruments can be sterilized by using a gas burner. However, the gas burner typically provides a turbulent flame, which may disturb the gas flow pattern within the operating zone of the cabinet.

### SUMMARY

An object of the invention is to provide a working area for a biosafety cabinet. An object of the invention is to provide a biosafety cabinet. An object of the invention is to provide a method for sterilizing an object.

According to an aspect, there is provided a biosafety cabinet (500), comprising:
- a cavity (110), which has an aperture (120), and
- one or more heating elements (HE1) arranged to heat an object (OBJ1) inserted into the cavity (110) in order to sterilize said object (OBJ1),
wherein the aperture (120) of the cavity (110) is located in the safe operating zone (ZONE1) of the cabinet (500), and the cavity (110) is attached to a wall (210) of the cabinet (500).

According to an aspect, there is provided s method for sterilizing an object (OBJ1) in a biosafety cabinet (500), the method comprising:
- inserting the object (OBJ1) into a cavity (110) through the aperture (120) of the cavity (110) in order to sterilize said object (OBJ1), wherein the cavity is attached to a wall (210) of the cabinet (500),
- heating the object (OBJ1) by one or more heating elements (HE1) when the object (OBJ1) is in the cavity (110), and
- moving the object (OBJ 1) from the cavity (110) to a working position outside the cavity (110) such that the object (OBJ1) remains within the safe operating zone (ZONE1) of the cabinet (500) during said moving.

Further aspects are defined in the claims.

The biosafety cabinet provides an enclosed ventilated workspace for safe working with a material, which is contaminated or potentially contaminated with a pathogen.

The user of the biosafety cabinet may sterilize an instrument during the work. The user may sterilize the instrument by inserting the instrument into the cavity of the sterilizing unit. The sterilizing unit may heat the instrument to a sufficient sterilization temperature. The sterilizing unit may comprise one or more heating elements, which may rapidly heat the instrument to a sufficient sterilization temperature e.g. by infrared radiation. The heating elements may be e.g. tungsten halogen lamps, and the lamps may emit infrared radiation to the instrument thorough the wall of the cavity.

The sterilizing unit may be attached e.g. to an inner wall of the cabinet such that the unit does not interfere with the air flow laminarity inside the working chamber. The sterilizing unit may be attached e.g. to an inner wall of the cabinet such that the working chamber will be easier to clean. Attaching the sterilizing unit to the wall may increase the free area on the working platform.

The aperture of the cavity may be positioned within the working zone of the cabinet such that the user does not need to move the instrument out of the working zone in order to sterilize the instrument. Yet, the sterilized instrument may remain in the working zone when it is removed from the cavity of the sterilizing unit and moved to a working position.

The aperture of the cavity may be positioned such that disturbance caused by the cavity to the flow pattern of the cabinet is small or negligible. In particular, the aperture of the cavity may be substantially flush with the back wall of the interior of the cabinet.

The position of the sterilizing cavity may be selected such that the cavity can be easily accessed by the user, while the risk of accidentally burning the skin of the user may be kept relatively low. Risk of explosion due to leaking combustible gas may be avoided.

### BRIEF DESCRIPTION OF THE DRAWINGS

In the following examples, several variations will be described in more detail with reference to the appended drawings, in which
- Fig. 1: shows, by way of example, in a cross-sectional side view, a biosafety cabinet, which comprises a sterilizing unit,
- Fig. 2: shows, by way of example, in a cross-sectional side view, air flows of the biosafety cabinet,
- Fig. 3a: shows, by way of example, in a cross-sectional side view, a sterilizing unit,
- Fig. 3b: shows, by way of example, in a cross-sectional side view, dimensions of the sterilizing unit,
- Fig. 4: shows, by way of example, in a cross-sectional side view, an object inserted in the cavity of the sterilizing unit, and
- Fig. 5: shows, by way of example, in a three dimensional view, a biosafety cabinet.

### DETAILED DESCRPTION

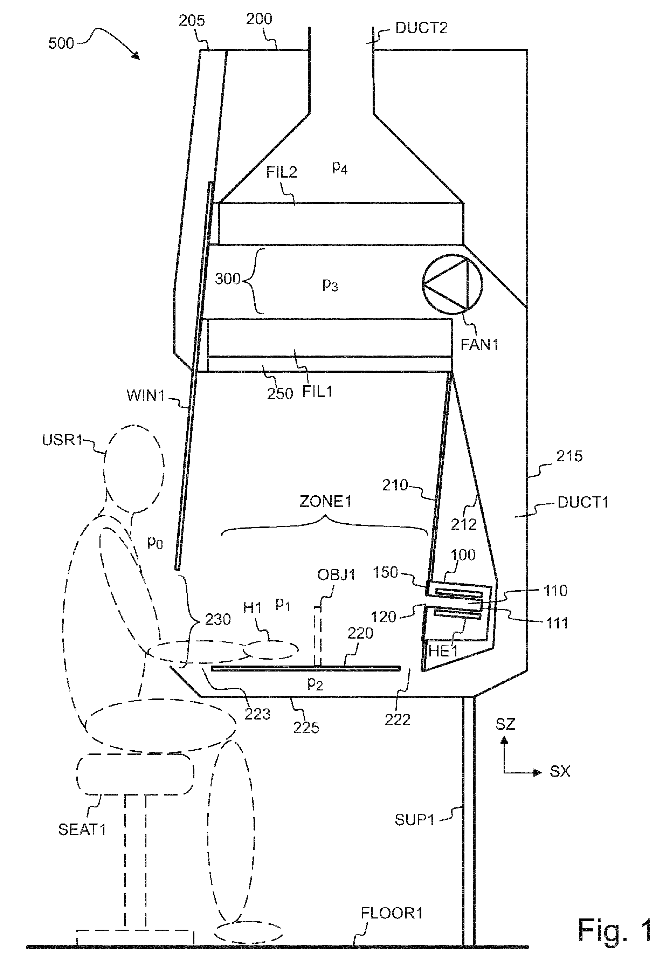

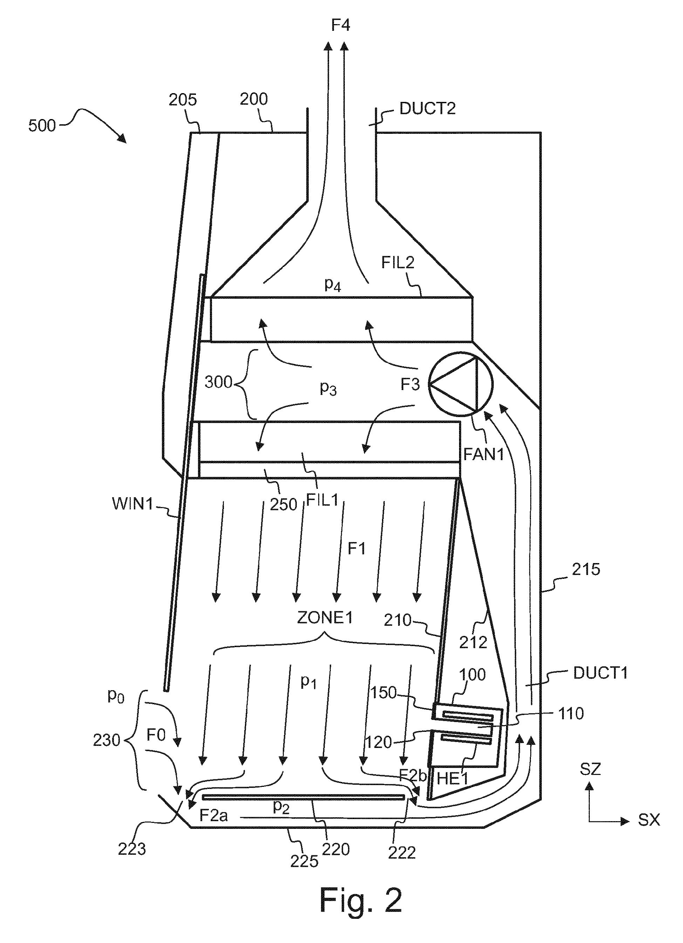

Referring to Fig. 1, a biosafety cabinet 500 may comprise a working platform 220, a front window WIN1, and a back wall 210a. The cabinet 500 may comprise a safe working zone ZONE1, which may be at least partly defined by the working platform 220, by a wall 210, and an air flow F1 of the cabinet 500 (See Fig. 2). The working platform 220, the front window WIN1, and the walls of the cabinet may define the working chamber of the cabinet 500.

An object OBJ1 may be contaminated or potentially contaminated with a pathogen. The object OBJ1 may be e.g. an empty test tube. The object OBJ1 may be e.g. a test tube which comprises a material. The object OBJ1 may be e.g. a laboratory instrument. The user USR1 may safely work with the object OBJ1 when the object is located in the safe working zone ZONE1, when the cabinet is operating normally.

The cabinet 500 may comprise a sterilizing unit 100 for sterilizing the object OBJ1. The sterilizing unit 100 may comprise a cavity 110, and one or more heating elements HE1. The one or more heating elements HE1 may be arranged to heat the object OBJ1 inserted in the cavity 110. The one or more heating elements HE1 may be arranged to sterilize the object OBJ1 by heating the OBJ1 to a temperature, which is higher than or equal to a sterilization temperature. Depending on the application, the sterilization temperature may be e.g. higher than or equal to 150 °C, higher than or equal to 200 °C, higher than or equal to 500°C, or even higher than or equal to 750 °C

The aperture of the cavity may be positioned within the working zone of the cabinet such that the user does not need to move the instrument out of the working zone in order to sterilize the instrument. Yet, the sterilized instrument may remain in the working zone when it is removed from the cavity of the sterilizing unit and moved to a working position.

The object OBJ1 may be sterilized by a method, which comprises:
- inserting the object into the cavity 110 through the aperture 120 of the cavity 110 in order to sterilize said object OBJ1, wherein the cavity is attached to a wall 210 of the cabinet 500,
- heating the object OBJ1 by one or more heating elements HE1 when the object OBJ1 is in the cavity 110, and
- moving the object OBJ1 from the cavity 110 to a working position outside the cavity 110 such that the object OBJ1 remains within the safe operating zone ZONE1 of the cabinet 500 during said moving.

The heating elements HE1 may heat the object OBJ1 e.g. by infrared radiation. The infrared radiation may be transmitted through the wall 111 of the cavity 110. The cavity 110 may have an aperture 120. The sterilizing unit 100 may optionally comprise a flow guide element 150. The sterilizing unit 100 may be enclosed in a housing 212.

The cabinet 500 may comprise an opening 230 for accessing the working zone ZONE1. The opening 230 may be partly defined by the bottom edge of a window WIN1. The cabinet 500 may comprise an opening 230 for inserting the object OBJ1 into the working zone ZONE1 and/or for removing an object OBJ1 from the working zone ZONE1. The user USR1 may also insert his hand H1, a tool, and/or a laboratory instrument to the working zone ZONE1 through the opening 230. The window WIN1 may be substantially planar and substantially transparent. The window may comprise e.g. glass, quartz, and/or plastic. The vertical position of the window WIN1 may be adjustable. The height of the opening 230 may be adjustable. The vertical position of the bottom edge of the window WIN1 may be adjustable e.g. in order to allow cleaning of the interior of the cabinet, to temporally facilitate manipulating the object in the working zone ZONE1, and/or to ensure sterile operating conditions in the working zone ZONE1. The window WIN1 may be slightly tilted with respect to the vertical direction SZ e.g. in order to increase the effective volume of the working zone ZONE1 and/or in order to provide a more ergonomic working position for the user USR1. An angle between the plane of the window WIN1 and the vertical direction SZ may be e.g. in the range of 0° to 60°, preferably in the range of 3° to 10°.

The user USR1 may be e.g. standing or seated on a seat SEAT1. The cabinet 500 may be supported on a base SUP1. The cabinet 500 may comprise a base SUP1. The base SUP1 may be positioned on the floor FLOOR1. The height of the base SUP1 may optionally be adjustable so that the upper edge of the viewing portion of the window WIN1 and the opening 230 may be set to a convenient height relative to the user USR1.

The cabinet 500 may comprise a front panel unit 205, which may be attached to a frame 200 of the cabinet 500.

The cabinet 500 may comprise one or more fans FAN1, and one or more filters FIL1, FIL2. The cabinet 500 may comprise a flow stabilization element 250. The cabinet 500 may optionally comprise one or more sensors to monitor the air flow rates and/or pressures of the cabinet.

SX, SY and SZ may denote orthogonal directions. The directions SX and SY may be horizontal. The direction SY may be substantially parallel to the window WIN1. The direction SZ may denote the vertical direction, i.e. the direction SZ may be opposite to the direction of gravity.

Fig. 2 shows, by way of example, the air flows F0, F1, F2a, F2b, F3, F4 of the cabinet during normal operation.

The cabinet 500 may comprise a first filter FIL1 for removing particles from an air flow F1. The cabinet may optionally comprise a stabilizing element 250 for stabilizing the air flow F1. The stabilizing element 250 may provide a substantially laminar air stream F1. The stabilizing element 250 may be e.g. a honeycomb element, which provides a laminar air stream F1.

The cavity 110 of the sterilizing unit 100 may be attached to the cabinet 500 such that aperture 120 of the cavity 110 is within the safe working zone ZONE1 of the cabinet 500. The cavity 110 may be attached e.g. to a wall of the interior of the cabinet. In particular, the cavity 110 may be attached to the back wall 210 of the interior of the cabinet 500. The cavity 110 may be defined by the wall 111 of the cavity.

The cabinet 500 may be arranged to operate such that the cavity 110 causes a small or minimum disturbance to the air flow F1. The cabinet 500 may be arranged to operate such that the wall 111 of the cavity 110 causes minimum disturbance to the air flow F1. In particular, the cavity 110 may be attached to the wall 210 of the cabinet such that the aperture 120 is substantially flush with the surface of the wall 210.

The cabinet 500 may comprise a fan FAN1, which may be arranged to move air F1 through the filter FIL1 and to suction air away from the working zone ZONE1. The air suctioned away from the working zone ZONE1 may be guided via a duct DUCT1 to the first filter FIL1. A first part F1 of the air removed from the working zone ZONE1 may be recirculated back to the working zone ZONE1 through the first filter FIL1. A second part F4 of the air removed from the working zone ZONE1 may be guided to a ventilation duct DUCT2.

The cabinet 500 may be arranged to provide an air flow F0 from the ambient room into the cabinet through the opening 230. The fan FAN1 may be arranged to provide the air flow F0 through the opening 230. The air flow F0 through the opening 230 may ensure that contaminated particles do not leak from the working zone ZONE1 to ambient air.

Air flows F2a, F2b may be guided from the working zone ZONE1 via inlets 222, 223. The fan FAN1 may be arranged to cause a pressure difference p₂-p₁ between the safe working zone ZONE1 and the bottom part of the cabinet 500. The pressure difference p₂-p₁ may cause an air flow F2a through an air inlet 223 and the pressure difference p₂-p₁ may cause an air flow F2b through an air inlet 222. The inlets 222, 223 may optionally comprise e.g. a perforated plate or a grill in order to prevent falling of the object OBJ1 to the bottom of the cabinet.

p₀ denotes air pressure of ambient air in the room. p₁ denotes air pressure in the working zone ZONE1. p₂ denotes air pressure below the bottom plate 220. p₃ denotes air pressure in a plenum chamber 300 between the filters FIL1, FIL2. p₄ denotes air flow in the ventilation duct DUCT2. The pressure difference p₁- p₀ may be negative in order to ensure proper air flow F0 into the cabinet through the opening 230. The pressure difference p₂-p₁ may be negative in order to suction air from the working zone ZONE1. The pressure difference p₃-p₁ may be positive in order to provide a sufficient air flow F1 to the working zone ZONE1 through the filter FIL1. The fan FAN1 may provide the air flow F3 into the plenum chamber 300. A first part of the air flow F3 may be guided to the working zone ZONE1 via the filter FIL1. A second part of the air flow F3 may be guided to the ventilation duct DUCT2 via the filter FIL2. The air flow F3 may be provided by the fan FAN1. The fan FAN1 may pump air from the duct DUCT1 to the chamber 300. The fan FAN1 may be e.g. a centrifugal fan. The cabinet may also comprise a first fan, which may be arranged to pump air from the duct DUCT1 via the filter FIL1 to the working zone ZONE1, and a second fan, which may be arranged to pump air from the duct DUCT1 via the filter FIL2 to the ventilation duct DUCT2.

In an embodiment, the air flow guided to the ventilation duct DUCT2 may be substantially equal to the air flow F0 through the opening 230. The pressure difference p₃-p₄ may be positive in order to ensure the proper air flow F0 into the cabinet 230 through the opening 230 and in order to provide the air flow from the cabinet to the ventilation duct DUCT2. The cabinet 500 may be located in a building, and the air flow F4 may be guided e.g. to the outside of the building by a ventilation system. The ventilation duct DUCT2 does not need to be a part of the cabinet 500.

The cabinet 500 may comprise a second filter FIL2 for removing particles from the air flow F4, which is guided to the ventilation duct DUCT2.

The filter FIL1 and/or FIL2 may be HEPA filters. HEPA is the acronym for High-efficiency particulate arrestance. The filter FIL1 and/or FIL2 may remove at least 99.97% of particles that have a size of 0.3 µm.

A flow guiding element 215 may at least partly define the air duct DUCT1. The cabinet 500 may comprise a bottom element 225.

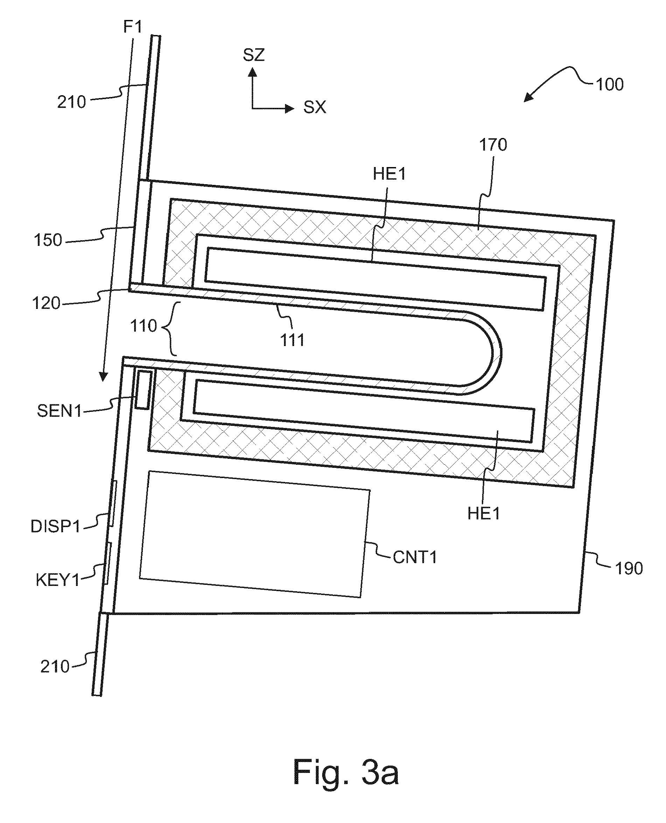

Fig. 3a shows, by way of example, a sterilizing unit 100, which comprises a cavity 110. The wall 111 of the cavity 110 may be made of a material, which is substantially transparent to infrared radiation (IR), and which can withstand elevated temperatures. The wall 111 may be made e.g. of quartz or sapphire. The wall 111 may comprise e.g. quartz or sapphire. The wall 111 may form a substantially leak tight structure so that the aperture 120 provides the only access to the cavity 110. Consequently, the cavity may remain sterile and/or the cavity does not leak pathogens into the inner parts of the cabinet 500. The wall 111 may form a tube, which is closed at one end.

The sterilizing unit 100 may be attached to the cabinet 500 such that the aperture 120 of the cavity 110 is within the safe working zone ZONE1 of the cabinet 500. The user USR1 may insert an object OBJ1 into the cavity via the aperture 120 in order to sterilize said object OBJ1. The sterilizing unit 100 may comprise one or more heating elements HE1. The heating elements HE1 may be electrical heating elements. The heating elements HE1 may be arranged to heat the object OBJ1 e.g. by infrared radiation (IR). The heating elements HE1 may emit infrared radiation (IR), which may be absorbed to the object OBJ1 in order to heat the object OBJ1 above a minimum sterilization temperature. A heating element HE1 may be e.g. a tungsten halogen lamp, ceramic heater, Metal rod heater, metal foil heater, metal coil heater. In particular, a tungsten halogen lamp may rapidly provide full heating power rapidly after it has been switched on. A heating element HE1 may also be e.g. a semiconductor laser.

The sterilizing unit 100 may optionally comprise a heat shield 170, to protect the surrounding structures from the heat. The heat shield 170 may comprise thermally insulating material. The heat shield 170 may comprise a reflective inner surface for reflecting infrared radiation towards the cavity 110.

The sterilizing unit 100 may optionally comprise a sensor SEN1. The sensor SEN1 may be a proximity sensor, which may be arranged to detect whether an object OBJ1 has been inserted in the cavity 110 or not. The sensor SEN1 may be e.g. an optical sensor, a capacitive sensor, or an inductive sensor. The sterilizing unit 100 may comprise several sensors having different operating principles. The sterilizing unit 100 may comprise a control unit CNT1. The control unit CNT1 may be configured to control operation of the heating elements HE1. The sterilizing unit 100 may be arranged to control operation of the heating elements HE1 based on a signal obtained from the proximity sensor SEN1.

The object OBJ1 may be sterilized by a method, which comprises:
- detecting when the object OBJ1 is inserted a cavity 110, and
- controlling heating of the object OBJ1 based on said detecting.

The object OBJ1 may be sterilized by a method, which comprises:
- detecting when the object OBJ1 is inserted a cavity 110, and
- starting and stopping heating of the object OBJ1 based on said detecting.

The sterilizing unit 100 may optionally comprise a sensor for monitoring the temperature of the object OBJ1.

The sterilizing unit 100 may optionally comprise e.g. one or more keys KEY1 for receiving user input from the user USR1. The sterilizing unit 100 may optionally comprise e.g. one or more indicators DISP1 for providing information to the user USR1. The indicator DISP1 may be e.g. a signal lamp or an alphanumeric display. The sterilizing unit 100 may optionally comprise a housing 190.

The sterilizing unit 100 may optionally comprise a timer for heating an object during a selectable time period, when the object OBJ1 has been inserted in the cavity 110.

The cavity 110 may be positioned with respect to the wall 210 of the cabinet 500 such that the cavity 110 causes small or minimum disturbance to the flow F1.

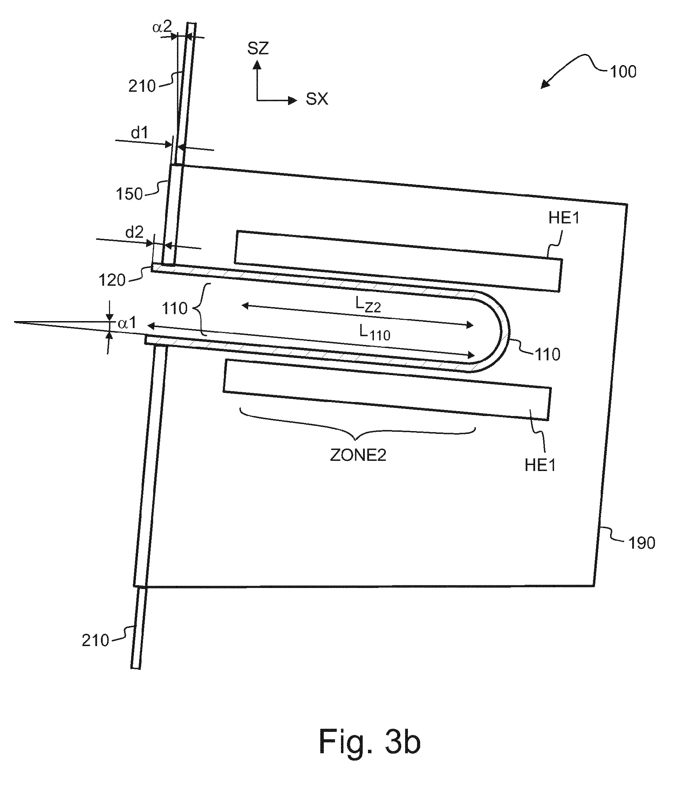

Referring to Fig. 3b, the cavity 110 may have an inner length L₁₀. The inner length L₁₀ may be e.g. in the range of 80 mm to 300 mm. The cavity 110 may have sterilization zone ZONE2. The length of the sterilization zone ZONE2 may be slightly smaller than the inner length L₁₁₀. The length of the sterilization zone ZONE2 may be e.g. in the range of 40 mm to 260 mm. The cross-section of the cavity may be e.g. circular or rectangular. In case of a substantially circular cavity, the inner diameter of the cavity 110 may be e.g. in the range of 10 mm to 40 mm.

The sterilizing unit 100 may optionally comprise a flow guiding element 150. The flow guide element 150 may a shape, which is arranged to cause only minimum resistance and/or turbulence to the air flow F1. The flow guide element 150 may have a streamlined shape. The flow guide element 150 may be e.g. a substantially planar plate, or a portion of a spherical or ellipsoidal surface. The flow guiding element 150 may protrude e.g. by a distance d1 with respect to the surface of the wall 210. The aperture 120 of the cavity 110 may protrude by a distance d2 with respect to the flow guiding element 150. The protrusion distance d2 may be e.g. smaller than 10 mm. The protrusion distance d2 may be e.g. smaller than 5 mm. The protrusion distance d2 may be e.g. smaller than 2 mm. The flow guiding element 150 may protrude with respect to the wall 210 by a distance d1, which is in the range of 0 to 50 mm, or the flow guiding element 150 may be recessed with respect to the wall 210 by a distance d1, which is in the range of 0 to 10 mm.

The cavity 110 may also be positioned such that the aperture 120 is substantially flush with the wall 210 and/or such that aperture 120 is substantially flush with the flow guiding element 150. The aperture 120 may protrude from the wall 210 by a distance d1+d2, which is in the range of 0 mm to 50 mm. The aperture 120 may protrude from the wall 210 by a distance d1+d2, which is in the range of 0 mm to 10 mm.

The cavity 110 may be directly attached to the wall 210 without using the flow guiding element 150. The aperture 120 may protrude with respect to the wall 210 by a distance, which is in the range of 0 to 50 mm. The aperture 120 may protrude with respect to the wall 210 by a distance, which is in the range of 0 to 10 mm.

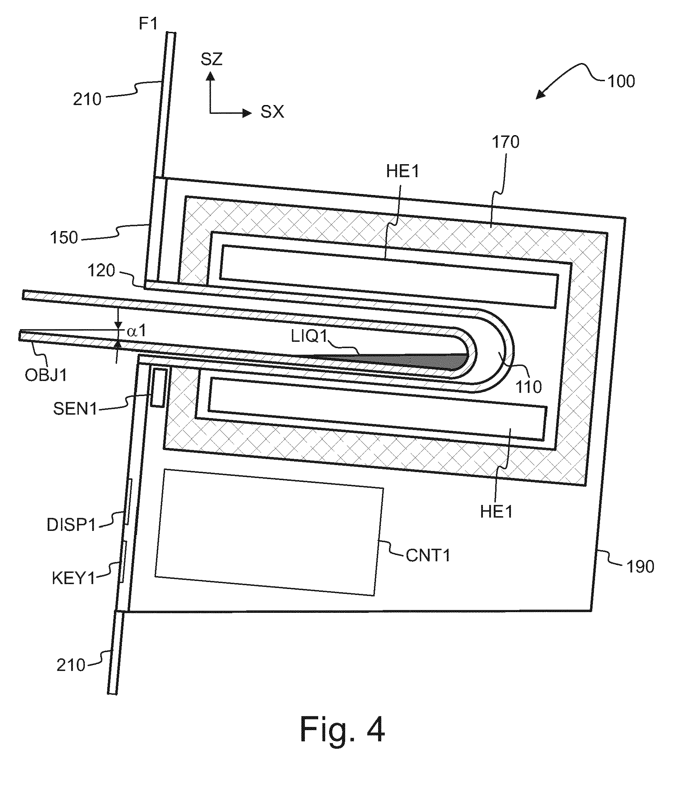

The lower internal surface of the cavity 110 may be inclined with respect to the horizontal direction SX e.g. in order to facilitate handling a test tube OBJ1, which contains a powder or a liquid LIQ1 (Fig. 4). The angle α1 between the lower internal surface of the cavity 110 and the horizontal direction SX may be e.g. in the range of 2° to 20°. The wall 210 of the cabinet 500 may be inclined with respect to the vertical direction SZ e.g. in order to facilitate the laminar or stable flow F1 in the vicinity of the wall 210. The angle α2 between the surface of the wall 210 and the vertical direction SZ may be e.g. in the range of 2° to 20°. In an embodiment, the angle α1 may be substantially equal to the angle α2. When the angle α1 is substantially equal to the angle α2, the aperture 120 may also be substantially perpendicular to the longitudinal direction of the cavity 110. This may facilitate manufacturing and/or installation of the cavity.

Heating by infrared radiation may allow rapid heating of the object OBJ1. Heating the object OBJ1 by the infrared radiation may save energy, as only minimum energy needs to be used for heating the cavity and/or the heating elements HE1 may consume energy only during the sterilizing operation.

The sterilizing unit 100 may also be arranged to heat the object OBJ1 by thermal conduction and/or by convection. The cavity 110 may be heated by using one or more heating elements HE1, and the hot wall of the cavity may subsequently transfer heat to the object OBJ1 by conduction and/or by convection. In an embodiment, the wall 111 of the cavity 110 may be arranged to operate as a heating element HE1. For example, the wall 111 may comprise an electrically conductive material, and the wall 111 may be heated e.g. by inductive heating.

The joint between the aperture 120 and wall 210 may be substantially leak tight, so as to minimize risk of contamination with pathogens. When using the flow guiding element 150, the joint between the aperture 120 and the flow guiding element 150 may be substantially leak tight. The cavity 110 may optionally be removable, e.g. in order to facilitate cleaning.

The sterilizing unit 100 may optionally comprise a cooling unit, e.g. a cooling fan for cooling the cavity 110 in the end of a sterilization operation. When using a cooling fan, the cooling air may be arranged to circulate so that the cooling air does not disturb the air flows of the working zone ZONE1.

Referring to Fig. 4, the lower internal surface of the cavity 110 may be inclined with respect to the horizontal direction SX e.g. in order to facilitate handling a test tube OBJ1, which contains a powder or a liquid LIQ1. The angle α1 between the lower internal surface of the cavity 110 and the horizontal direction SX may be e.g. in the range of 2° to 20°.

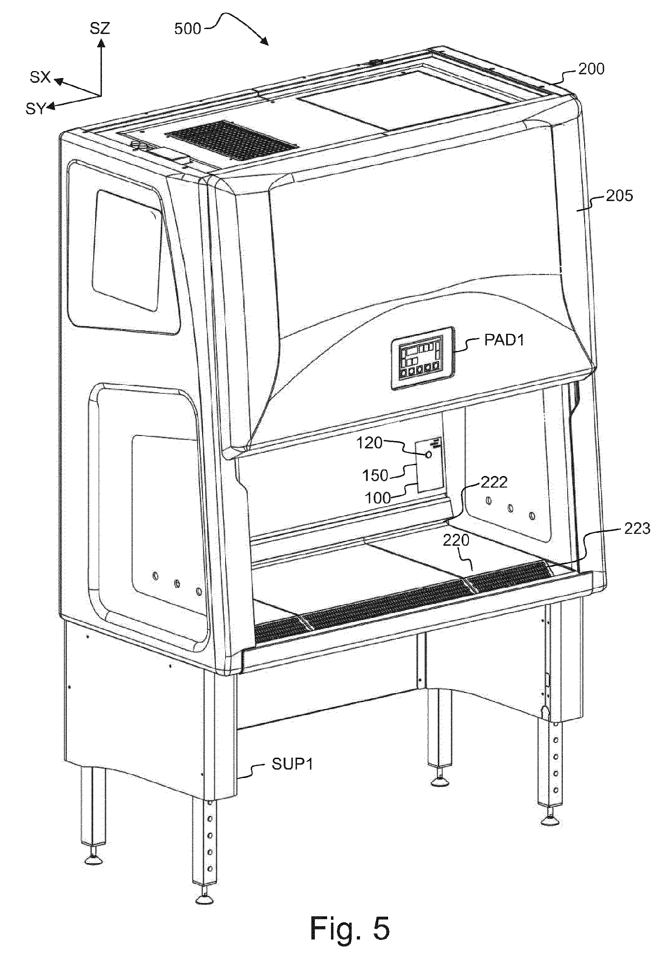

Referring to Fig. 5, the sterilizing unit may be placed e.g. to the left or right side of the cabinet. The aperture 120 of the cavity 110 may be positioned e.g. at the right side of the back wall 210 or at the left side of the back wall 210, e.g. in order to facilitate the sterilizing operation in a situation where a plurality of objects are positioned at the central area of the working zone ZONE1

Visual inspection of cavity may be needed e.g. for checking whether cavity needs cleaning and/or when cleaning the cavity. The user USR1 may conveniently see the interior of the cavity 110 when the cavity 110 is attached to the back wall 210 of the cabinet 500. The user USR1 may conveniently see the interior of the cavity 110 without putting his head into the working chamber or without a need to use a visual inspection mirror.

The cabinet 500 may optionally comprise a user interface for receiving user input from the user USR1 and for displaying information to the user USR1. The cabinet 500 may optionally comprise an input unit PAD1 for obtaining user input from the user USR1. The input unit PAD1 may comprise e.g. a touch screen, a keypad, and/or a mouse. In particular, a touch screen may be attached to a front panel unit 205 of the cabinet 500.

In an embodiment, the sterilizing unit 100 may be operated by receiving user input from the user USR1 to the sterilizing unit 100 via the touch screen PAD1, so that the user does not need to touch the sterilizing unit 100 with his hand.

The cabinet 500 may be a class II biosafety cabinet, as determined by the NSF International Standard 49. The biosafety cabinet may also be called as the microbiological safety cabinet.

The safe working area ZONE1 of the cabinet 500 may mean the region, which includes all those positions where an object is not contaminated by impurities of the ambient room air and where said object does not contaminate the ambient room air.

For the person skilled in the art, it will be clear that modifications and variations of the devices and the methods according to the present invention are perceivable. The figures are schematic. The particular embodiments described above with reference to the accompanying drawings are illustrative only and not meant to limit the scope of the invention, which is defined by the appended claims.

## Claims

1. A biosafety cabinet (500), comprising:
- a cavity (110), which has an aperture (120), and
- one or more heating elements (HE1) arranged to heat an object (OBJ1) inserted into the cavity (110) in order to sterilize said object (OBJ1),
wherein the aperture (120) of the cavity (110) is located in the safe operating zone (ZONE1) of the cabinet (500), and the cavity (110) is attached to a wall (210) of the cabinet (500).

2. The cabinet (500) of claim 1, wherein the aperture (120) protrudes with respect to the wall (210) by a distance (d1+d2), which is in the range of 0 to 50 mm.

3. The cabinet (500) of claim 1 or 2, wherein the aperture (120) protrudes with respect to the wall (210) by a distance (d1+d2), which is in the range of 0 to 10 mm.

4. The cabinet (500) according to any of the claims 1 to 3, wherein the angle α1 between the lower internal surface of the cavity 110 and the horizontal direction SX is in the range of 2° to 20°.

5. The cabinet (500) according to any of the claims 1 to 4, wherein the angle α2 between the surface of the wall 210 and the vertical direction SZ is in the range of 2° to 20°.

6. The cabinet (500) according to any of the claims 1 to 5, wherein the heating elements (HE1) are arranged to heat the object (OBJ1) by infrared radiation (IR1) transmitted through the wall (111) of the cavity (110).

7. The cabinet (500) according to any of the claims 1 to 6, comprising a sensor (SEN1), which is arranged to arranged to detect whether an object OBJ1 is inserted in the cavity (110), and a control unit CNT1, which is configured to control the heating elements (HE1) based on a signal obtained from the sensor (SEN1).

8. The cabinet (500) according to any of the claims 1 to 7, comprising a timer to control the duration of a heating period.

9. A method for sterilizing an object (OBJ1) in a biosafety cabinet (500), the method comprising:
- inserting the object (OBJ1) into a cavity (110) through the aperture (120) of the cavity (110) in order to sterilize said object (OBJ1), wherein the cavity is attached to a wall (210) of the cabinet (500),
- heating the object (OBJ1) by one or more heating elements (HE1) when the object (OBJ1) is in the cavity (110), and
- moving the object (OBJ 1) from the cavity (110) to a working position outside the cavity (110) such that the object (OBJ1) remains within the safe operating zone (ZONE1) of the cabinet (500) during said moving.

10. The method of claim 9, wherein the aperture (120) protrudes with respect to the wall (210) by a distance (d1+d2), which is in the range of 0 to 50 mm.

11. The method of claim 9 or 10, wherein the aperture (120) protrudes with respect to the wall (210) by a distance (d1+d2), which is in the range of 0 to 10 mm.

12. The method according to any of the claims 9 to 11, wherein the object (OBJ1) is a test tube, and the angle α1 between the lower internal surface of the cavity 110 and the horizontal direction SX is in the range of 2° to 20°.

13. The method according to any of the claims 9 to 12, wherein the angle α2 between the surface of the wall 210 and the vertical direction SZ is in the range of 2° to 20°.

14. The method according to any of the claims 9 to 13, wherein the object (OBJ1) is heated by infrared radiation (IR1) transmitted through the wall of the cavity (110).

15. The method according to any of the claims 9 to 14, comprising:
- detecting when the object (OBJ1) is inserted a cavity (110), and
- starting and stopping said heating based on said detecting.
